# EUROPÄISCHE PATENTSCHRIFT

(11) **EP 1 752 118 B1**
(45) Veröffentlichungstag und Bekanntmachung des Hinweises auf die Patenterteilung: **05.05.2010**
(21) Anmeldenummer: 05017647.8
(22) Anmeldetag: 12.08.2005
(51) Int. Cl.: A61F 9/013

(54) **Mikrochirurgische Schneidanordnung, insbesondere für die refraktive Augenchirurgie**
Microsurgical cutting assembly, in particular for eye surgery
Dispositif de coupe microchirurgical, en particulier pour chirurgie oculaire

(43) Veröffentlichungstag der Anmeldung: 14.02.2007
(73) Patentinhaber: WaveLight AG, 91058 Erlangen (DE)
(72) Erfinder: Jeglorz, Tobias, 90425 Nürnberg (DE); Donitzky, Christof, 90542 Eckental (DE)
(74) Vertreter: von Hellfeld, Axel

(56) Entgegenhaltungen:
- WO-A-01/91650
- WO-A-01/97729
- WO-A-2004/060222
- US-A- 2 679 100
- US-A- 5 215 104

## Beschreibung

Die Erfindung betrifft ein Mikrokeratom, insbesondere für die refraktive Chirurgie in der Augenheilkunde, mit einem Schneidklingenhalter und einer in eine Aufnahme des Schneidklingenhalters lateral beweglich einsetzbaren Schneidklingeneinheit, wobei die Schneidklingeneinheit eine Schneidklinge mit einer an einem ersten Klingenrand gebildeten Schneidkante aufweist, wobei der Schneidklingenhalter lineare Anlagemittel aufweist, an welchen sich die Schneidklingeneinheit in ordnungsgemäß in die Aufnahme eingesetztem Zustand über einen dem ersten Klingenrand gegenüberliegenden zweiten Klingenrand der Schneidklinge lateral beweglich abstützt.

Zur refraktiven Sehfehlerbehandlung des menschlichen Auges ist es bekannt, mittels eines als Mikrokeratom bezeichneten mikrochirurgischen Schneidinstruments ein oberflächliches Scheibchen (Flap) von der Hornhaut so abzulösen, dass es an einer Seite an einem sogenannten Hinge noch mit der Hornhaut verbunden ist. Durch Anheben und Wegklappen des Flaps werden die darunterliegenden Hornhautbereiche (Stroma) zur Hornhautneuformung mittels eines Lasers zugänglich. Nach Beendigung der Laserbehandlung wird der Flap zurückgeklappt.

Das Mikrokeratom weist üblicherweise eine auf den Augapfel (Limbus) aufzusetzende Saugringeinheit auf, an der ein mit einer Schneidklinge auswechselbar bestückter Schneidklingenhalter beweglich geführt ist. Zur Flappräparation wird der Schneidklingenhalter mittels eines elektromotorischen Antriebs in einer Vorschubrichtung über die Hornhaut bewegt. Dabei schneidet die mit ihrer Schneidkante aus dem Schneidklingenhalter vorstehende Schneidklinge in die Hornhaut ein und löst den Flap ab. Zusätzlich zum Vorschub des Schneidklingenhalters wird üblicherweise die Schneidklinge in laterale Oszillation versetzt.

Bei einer bekannten Ausbildung stützt sich die Schneidklinge mit ihrem der Schneidkante gegenüberliegenden hinteren Klingenrand an dem Schneidklingenhalter ab. Der hintere Klingenrand ist dabei als gerade Anlagekante ausgebildet. Für eine präzise Führung der Schneidklinge bei ihrer oszillierenden Seitwärtsbewegung ist eine hinreichende Länge der rückwärtigen Anlagekante erforderlich. Dies geht einher mit einer entsprechend erhöhten Reibung der Schneidklinge an dem Schneidklingenhalter. Angesichts einer Oszillationsfrequenz der Schneidklinge von oftmals zwischen 15 und 500 Hz, insbesondere zwischen 100 und 250 Hz ist freilich eine hohe Leichtgängigkeit der Schneidklinge in dem Schneidklingenhalter sehr wünschenswert. Bei solchen Oszillationsfrequenzen ist auch das Gewicht der Schneidklinge ein wichtiger Faktor. Es sollte möglichst klein sein, um insgesamt nur geringe zu bewegende Massen zu haben.

US 5,215,104 zeigt ein Mikrokeratom für die refraktive Augenchirurgie mit einem Klingenhalter zur oszillierenden Aufnahme einer Schneidklinge.

Aufgabe der Erfindung ist es, ein Mikrokeratom der eingangs bezeichneten Art zu schaffen, welches eine leichtgängige und gleichzeitig präzise Führung der Schneidklinge in dem Schneidklingenhalter ermöglicht.

Zur Lösung dieser Aufgabe ist erfindungsgemäß vorgesehen, dass der zweite Klingenrand der Schneidklinge mindestens zwei im Abstand voneinander angeordnete Anlagestellen für die Anlage der Schneidklinge an den Anlagemitteln des Schneidklingenhalters bildet und zwischen jedem Paar einander benachbarter Anlagestellen gegenüber einer die betreffenden Anlagestellen verbindenden gedachten Geraden in Richtung zum ersten Klingenrand zurückversetzt ist. Durch die Vorsehung mehrerer im Abstand voneinander angeordneter Anlagestellen am zweiten (hinteren) Klingenrand kann unter Beibehaltung einer präzisen Führung der Schneidklinge die Größe des Kontaktbereichs, in dem der hintere Klingenrand mit den Anlagemitteln des Schneidklingenhalters in Eingriff gelangt, insgesamt klein gehalten werden. Dies verringert die Reibung zwischen Schneidklinge und Schneidklingenhalter. Der Bereich zwischen den Anlagestellen kann vorteilhaft zur Material- und somit Gewichtsreduzierung der Schneidklinge genutzt werden, indem dort der hintere Klingenrand zum Klingeninneren hin zurückversetzt ist.

Bei gattungsgemäßen Schneidanordnungen bestimmt der Überstand der Schneidkante der Schneidklinge über den Schneidklingenhalter die Dicke des abgelösten Hornhautflaps. Der Klingenüberstand hängt seinerseits von der Länge der Schneidklinge von der Schneidkante bis dorthin ab, wo sich die Klinge nach hinten abstützt, also bis zum hinteren Klingenrand. Da es für den Operationserfolg von großer Wichtigkeit ist, dass eine vorgegebene, gewünschte Flapdicke präzise eingehalten wird, werden an die Genauigkeit der Klingenlänge üblicherweise sehr hohe Anforderungen gestellt. Bei einer einzigen Anlagekante, die sich über einen Großteil der Klingenbreite erstreckt, können Ungleichmäßigkeiten in der Geradheit der Anlagekante sowie eine gewisse Aparallelität der Anlagekante zur Schneidkante nicht völlig ausgeschlossen werden. Dies kann zu Abweichungen der tatsächlichen von der gewünschten Flapdicke führen. Die Vorsehung von getrennten, lokal begrenzten Anlagestellen statt einer einzigen durchgehenden Anlagekante ist dann für die Einhaltung der geforderten geringen Toleranzen der effektiven Klingenlänge vorteilhaft.

Bei einer bevorzugten Ausführungsform ist mindestens eine Anlagestelle der Schneidklinge von einem gerundeten Abschnitt des zweiten Klingenrands gebildet. Alternativ oder zusätzlich kann mindestens eine Anlagestelle der Schneidklinge von einem spitz zulaufenden Abschnitt des zweiten Klingenrands gebildet sein. Mit einem rundlichen oder spitz zulaufenden Verlauf des betreffenden Klingenrandabschnitts kann eine Anlagestelle geschaffen werden, die einen nahezu punktuellen Kontakt mit den Anlagemitteln des Schneidklingenhalters hat. Auf diese Weise kann eine äußerst hohe Führungsgenauigkeit der Schneidklinge für die Seitwärtsoszillation erhalten werden. Es ist aber auch vorstellbar, mindestens eine Anlagestelle der Schneidklinge von einem geradlinig verlaufenden Abschnitt des zweiten Klingenrands zu bilden. Eine solche geradlinige Anlagestelle kann im Vergleich zu einer einzigen, sich über einen Großteil der Klingenbreite erstreckenden geraden Anlagekante viel kürzer sein und deswegen beträchtlich weniger anfällig für etwaige Ungleichmäßigkeiten in der Geradheit und Aparallelitäten zur Schneidkante sein.

Es können alle Anlagestellen des hinteren Klingenrands die gleiche Grundgeometrie besitzen, also beispielsweise rundlich oder spitz zulaufend. Es können aber auch mindestens zwei Anlagestellen unterschiedliche Geometrie besitzen. Dies ist insbesondere dann denkbar, wenn der zweite Klingenrand der Schneidklinge mindestens drei Anlagestellen bildet. Dann können zwei äußere Anlagestellen die gleiche Grundgeometrie besitzen und eine mittlere Anlagestelle eine andere Geometrie. Es ist grundsätzlich auch denkbar, alle Anlagestellen des hinteren Klingenrands mit unterschiedlicher Geometrie auszuführen.

Für eine exakte Positionierung der Schneidklingeneinheit empfiehlt es sich, wenn bei ordnungsgemäß in den Schneidklingenhalter eingesetzter Schneidklingeneinheit zwischen dieser und dem Schneidklingenhalter federelastische Vorspannmittel wirksam sind, welche ein Andrücken der Schneidklinge an ihren Anlagestellen gegen die Anlagemittel des Schneidklingenhalters bewirken.

Für eine reibungsarme Führung der Schneidklinge an den Anlagemitteln des Schneidklingenhalters ist eine bei Betrachtung in einem Schnitt quer zur Linearerstreckung der Anlagemittel bogenartig, insbesondere kreisbogenartig, gekrümmte Kontur der Anlagemittel vorteilhaft.

Die Erfindung wird nachfolgend anhand der beigefügten Zeichnungen weiter erläutert. Es stellen dar:
Figur 1 einen Schnitt durch einen Klingenaufnahmebereich eines Schneidklingenhalters gemäß einem Ausführungsbeispiel,
Figur 2 perspektivisch ein Ausführungsbeispiel einer zur Verwendung mit dem Schneidklingenhalter der Figur 1 geeigneten Schneidklingeneinheit und
Figuren 3 bis 6 verschiedene Varianten einer Schneidklinge für die Schneidklingeneinheit der Figur 2.

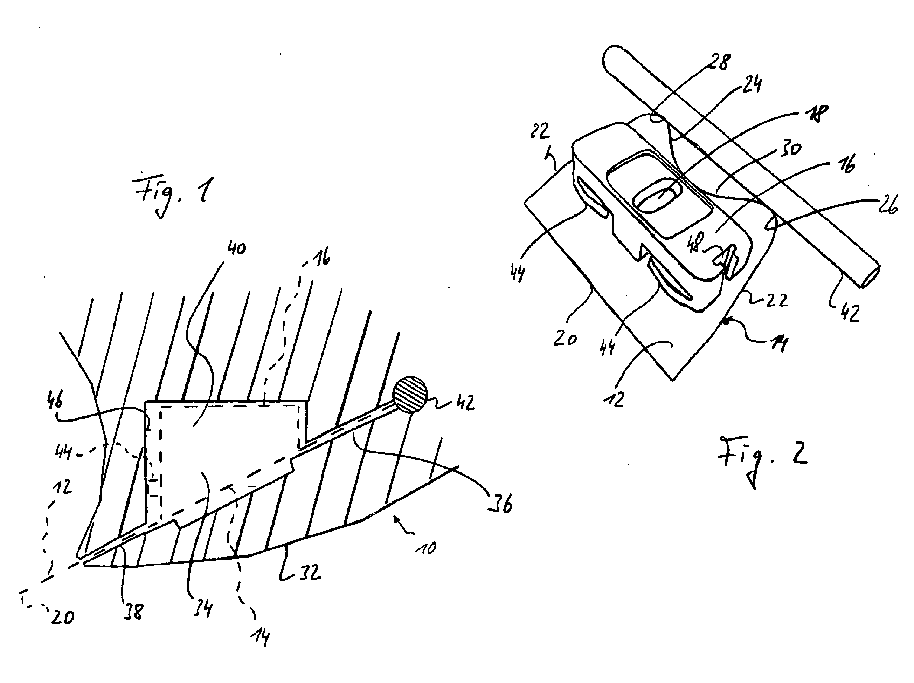

Der in Figur 1 ausschnittsweise gezeigt Schneidklingenhalter - allgemein mit 10 bezeichnet - ist in an sich bekannter Weise an einer nicht näher dargestellten Saugringeinheit eines augenchirurgischen Mikrokeratoms beweglich geführt gehalten bzw. halterbar. Mittels eines ebenfalls nicht näher dargestellten elektromotorischen Antriebs ist der Schneidklingenhalter, nachdem die Saugringeinheit auf ein zu operierendes Auge aufgesetzt und dort mittels Vakuum angesaugt wurde, in einer Vorschubrichtung über die Hornhaut des Auges bewegbar, wobei eine Schneidklinge 12 (Figur 2) einen Flap von der Hornhaut abtrennt.

Wie in Figur 2 erkennbar, ist die Schneidklinge 12 Teil einer Schneidklingeneinheit 14, welche zusätzlich zur eigentlichen Schneidklinge 12 einen Aufsatz 16 an einer der flachen Oberseiten der Schneidklinge 12 umfasst. Der Aufsatz 16 ist fest mit der Schneidklinge 12 verbunden, vorzugsweise durch eine formschlüssige oder kraftschlüssige Verbindung. Eine stoffschlüssige Verbindung unter Verwendung eines Klebstoffs ist grundsätzlich auch vorstellbar. Der Aufsatz 16 vereinfacht die Handhabung der Schneidklingeneinheit 14. An seiner freiliegenden Oberseite weist er eine längliche Vertiefung 18 auf, in welche im Betrieb des Mikrokeratoms ein Exzenterzapfen einer Abtriebswelle des erwähnten elektromotorischen Antriebs eingreift. Die Schneidklingeneinheit 14 wird so in seitlich oszilierende Bewegung (quer zur Vorschubrichtung) versetzt.

Die Schneidklinge 12 weist einen geradlinigen vorderen Klingenrand auf, der eine Schneidkante 20 bildet. Stumpfe seitliche Klingenränder 22 schließen an den vorderen Klingenrand an und gehen im rückwärtigen Bereich der Schneidklinge 12 in einen hinteren Klingenrand 24 über. Der hintere Klingenrand 24 ist mit zwei im Abstand voneinander angeordneten, rundlichen Anlageabschnitten 26, 28 ausgeführt, zwischen denen ein zurückversetzter Klingenrandabschnitt 30 vorhanden ist. Der Krümmungsverlauf der rundlichen Klingenrandabschnitte 26, 28 kann beispielsweise zumindest stückweise kreisbogenförmig sein. Der zurückversetzte Klingenrandabschnitt 30 ist im gezeigten Beispielfall ebenfalls rundlich ausgeführt, kann aber auch einen beliebigen anderen Verlauf besitzen. Der durch den zurückversetzten Klingenrandabschnitt 30 entstandene Kontureinschnitt der Schneidklinge 12 reicht vorzugsweise nicht weiter als bis zu dem Aufsatz 16.

Der Schneidklingenhalter 10 weist ein Klingenhaltergehäuse 32 auf, in dem eine Aufnahme 34 für die Schneidklingeneinheit 14 ausgebildet ist. Die Aufnahme 34 ist zweckmäßigerweise zu einer Seite des Schneidklingenhalters 10 hin offen, sodass die Schneidklingeneinheit 14 seitlich in die Aufnahme 34 des Schneidklingenhalters 10 eingeschoben und nach Gebrauch wieder herausgezogen werden kann. Die Aufnahme 34 besitzt zwei schlitzförmige Abschnitte 36, 38, zwischen denen ein aufgeweiteter Abschnitt 40 vorhanden ist. Der Aufsatz 16 gelangt beim Einführen der Schneidklingeneinheit 14 in die Aufnahme 34 in den aufgeweiteten Abschnitt 40, während die Klingenbereiche vor und hinter im Aufsatz 16 in die schlitzförmigen Abschnitte 36, 38 der Aufnahme 34 gelangen. Ist die Schneidklingeneinheit 14 ordnungsgemäß in die Aufnahme 34 eingesetzt, wie gestrichelt in Figur 1 erkennbar, so steht die Schneidklinge 12 mit ihrer Schneidkante 20 aus dem Schneidklingenhalter 10 vor. Gleichzeitig stützt sich die Schneidklinge 12 mit ihren rückwärtigen Anlageabschnitten 26, 28 an einem in das Klingenhaltergehäuse 32 eingebauten Führungsanlagestab 42 ab.

Aufgrund der rundlichen Ausbildung der Anlageabschnitte 26, 28 ist der Kontakt zwischen der Schneidklinge 12 und dem Führungsanlagestab 42 nahezu punktuell, d.h. es gibt insgesamt zwei Anlagepunkte zwischen der Schneidklinge 12 und dem Führungsanlagestab 42. Die punktuelle Abstützung der Schneidklinge 12 am Führungsanlagestab 42 sorgt für eine besonders hohe Reibungsarmut, wenn der Schneidklingenhalter 14 im Betrieb des Mikrokeratoms in seitliche Oszillation versetzt wird und sich dabei die Anlageabschnitte 26, 28 entlang des geradlinigen Führungsanlagestabs 42 bewegen. Der punktuelle Kontakt wird durch eine bogenförmig gekrümmte Außenumfangsfläche des Führungsanlagestabs 42 noch befördert. Zweckmäßigerweise ist der Führungsanlagestab 42 deswegen von einem Stab mit Kreisquerschnitt gebildet, wie insbesondere in Figur 1 erkennbar. Es versteht sich, dass alternativ Stäbe mit anderen Querschnittsformen verwendet werden können, beispielsweise ein Stab mit ovalem oder elliptischem Querschnitt. Natürlich können auch Stäbe, die eine ebene Anlagefläche für die Schneidklinge 12 bereitstellen, verwendet werden, so etwa ein Stab mit Rechteck- oder Dreieck-Querschnitt.

In Figur 2 ist zu erkennen, dass der Aufsatz 16 mit zwei Federzungen 44 ausgeführt ist, welche zur Zusammenwirkung mit einer vorderen Begrenzungswand 46 des aufgeweiteten Abschnitts 40 der Aufnahme 34 bestimmt und ausgebildet sind. Die Federzungen 44 bewirken eine Vorspannung auf die Schneidklingeneinheit 14 in Richtung nach hinten, d.h. gegen den Führungsanlagestab 42, wenn die Schneidklingeneinheit 14 ordnungsgemäß in die Aufnahme 34 eingesetzt ist. Die Federzungen 44 können einstückig mit dem Aufsatz 16 hergestellt sein, sie können jedoch alternativ von gesonderten Elementen gebildet sein, die an den Aufsatz 16 angeklebt oder angeschweißt oder anderweitig daran befestigt sind. Es versteht sich, dass zur Erzeugung einer Federvorspannung, die die Schneidklingeneinheit 14 in Richtung auf den Führungsanlagestab 42 vorspannt, beliebig gestaltete Federelemente verwendet werden können. Es ist im übrigen auch nicht notwendig, dass - wie im Beispielfall der Figuren 1 und 2 - die Federelemente (hier die Federzungen 44) an der Schneidklingeneinheit 44 angeordnet sind. Sie können genauso an dem Schneidklingenhalter 10 in der Aufnahme 34 oder in diese hineinragend angeordnet sein.

Seitlich in den Aufsatz 16, der beispielsweise aus Kunststoff spritzgegossen sein kann, ggf. aber auch aus Metall oder einem keramischen Werkstoff gefertigt sein kann, ist eine hinterschnittene T-Nut 48 eingearbeitet, mit welcher eine nicht näher dargestellte Betätigungsstange in schub- und zugkraftübertragenden Eingriff gebracht werden kann. Mittels einer solchen Betätigungsstange kann die Schneidklingeneinheit 14 problemlos in die Aufnahme 14 eingeschoben und/oder aus dieser herausgezogen werden.

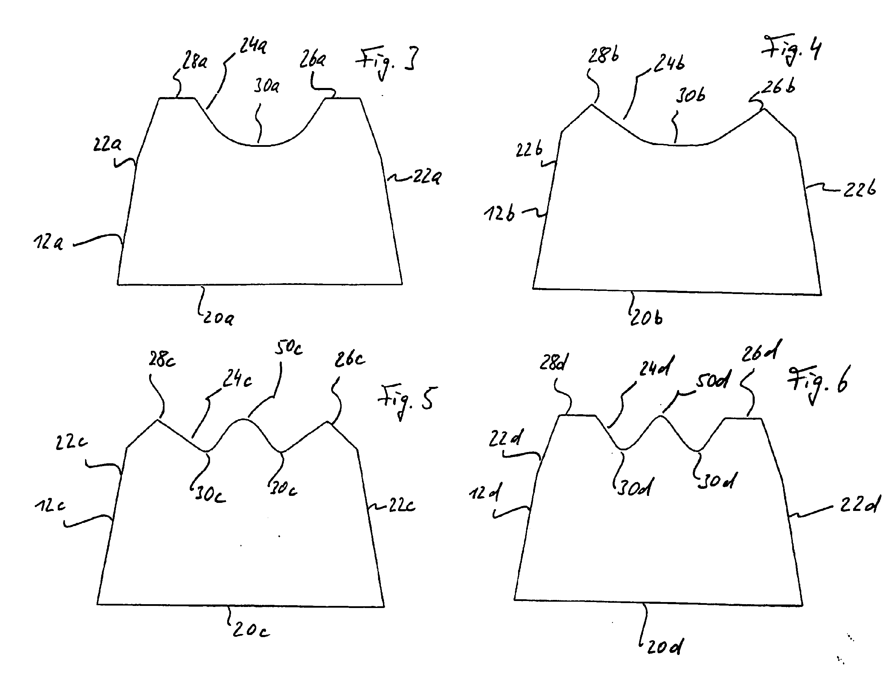

Es wird nun auf die in den Figuren 3 bis 6 gezeigten alternativen Klingenkonturen der Schneidklinge verwiesen. In diesen Figuren sind gleichwirkende Komponenten mit gleichen Bezugszeichen wie in Figur 2 bezeichnet, jedoch ergänzt durch einen Kleinbuchstaben.

Bei der Variante der Figur 3 sind die Anlageabschnitte 26a, 28a der Schneidklinge 12a von kurzen, geradlinigen Segmenten des hinteren Klingenrands 24a gebildet.

Bei der Variante der Figur 4 sind dagegen die Anlageabschnitte 26b, 28b der Schneidklinge 12b spitz zulaufend ausgeführt, sodass sie ähnlich wie die rundlichen Anlageabschnitte 26, 28 der in Figur 2 gezeigten Schneidklinge 12 einen im Wesentlichen punktuellen Kontakt mit einer in der Klingenaufnahme des Schneidklingenhalters vorgesehenen rückwärtigen Widerlagerfläche herstellen können.

Bei der Variante der Figur 5 ist zusätzlich zu den beiden - hier wiederum spitz zulaufenden - Anlageabschnitten 26c, 28c noch ein dritter, mittiger Anlageabschnitt 50c vorgesehen, der jedoch mit einer anderen Geometrie als die beiden äußeren Anlageabschnitte 26c, 28c ausgeführt ist. Konkret ist im Beispielfall der Figur 5 der mittlere Anlageabschnitt 50c rundlich ausgeführt, sodass er wie die beiden äußeren Anlageabschnitte 26c, 28c einen annähernden Punktkontakt mit der rückwärtigen Widerlagerfläche des Schneidklingenhalters herstellt. Beidseits des mittleren Anlageabschnitts 50c ist je ein zurückversetzter Abschnitt 30c vorgesehen.

Die Variante der Figur 6 unterscheidet sich von derjenigen der Figur 5 dadurch, dass die beiden äußeren Anlageabschnitte 26d, 28d ähnlich wie in Figur 3 als kurze, geradlinige Klingenrandsegmente ausgebildet sind.

Ungeachtet der konkreten Geometrie der Anlageabschnitte liegen bei den beiden in den Figuren 5 und 6 gezeigten Varianten die Kontaktpunkte bzw. -bereiche aller Anlageabschnitte auf einer gedachten Geraden, sodass eine präzise Seitwärtsführung der Schneidklinge gewährleistet ist.

## Patentansprüche

1. Mikrokeratom, insbesondere für die refraktive Augenchirurgie, mit einem Schneidklingenhalter (10) und einer in eine Aufnahme (34) des Schneidklingenhalters lateral beweglich einsetzbaren Schneidklingeneinheit (14), wobei die Schneidklingeneinheit eine Schneidklinge (12) mit einer an einem ersten Klingenrand gebildeten Schneidkante (20) aufweist, wobei der Schneidklingenhalter lineare Anlagemittel (42) aufweist, an welchen sich die Schneidklingeneinheit in ordnungsgemäß in die Aufnahme eingesetztem Zustand über einen dem ersten Klingenrand gegenüberliegenden zweiten Klingenrand (24) der Schneidklinge lateral beweglich abstützt,
**dadurch gekennzeichnet, dass** der zweite Klingenrand der Schneidklinge mindestens zwei im Abstand voneinander angeordnete Anlagestellen (26, 28) für die Anlage der Schneidklinge an den Anlagemitteln des Schneidklingenhalters bildet und zwischen jedem Paar einander benachbarter Anlagestellen gegenüber einer die betreffenden Anlagestellen verbindenden gedachten Geraden in Richtung zum ersten Klingenrand zurückversetzt (bei 30) ist.

2. Mikrokeratom nach Anspruch 1,
**dadurch gekennzeichnet, dass** mindestens eine Anlagestelle (26, 28) der Schneidklinge (12) von einem gerundeten Abschnitt des zweiten Klingenrands (24) gebildet ist.

3. Mikrokeratom nach Anspruch 1 oder 2,
**dadurch gekennzeichnet, dass** mindestens eine Anlagestelle (26b, 28b) der Schneidklinge (12b) von einem spitz zulaufenden Abschnitt des zweiten Klingenrands (24b) gebildet ist.

4. Mikrokeratom nach einem der vorhergehenden Ansprüche,
**dadurch gekennzeichnet, dass** mindestens eine Anlagestelle (26a, 28a) der Schneidklinge (12a) von einem geradlinig verlaufenden Abschnitt des zweiten Klingenrands (24a) gebildet ist.

5. Mikrokeratom nach einem der vorhergehenden Ansprüche,
**dadurch gekennzeichnet, dass** mindestens zwei Anlagestellen (26c, 50c) unterschiedliche Geometrie besitzen.

6. Mikrokeratom nach einem der vorhergehenden Ansprüche,
**dadurch gekennzeichnet, dass** der zweite Klingenrand (24c) der Schneidklinge (12c) mindestens drei Anlagestellen (26c, 28c, 50c) bildet.

7. Mikrokeratom nach einem der vorhergehenden Ansprüche,
**dadurch gekennzeichnet, dass** bei ordnungsgemäß in den Schneidklingenhalter (10) eingesetzter Schneidklingeneinheit (14) zwischen dieser und dem Schneidklingenhalter federelastische Vorspannmittel (44) wirksam sind, welche ein Andrücken der Schneidklinge an ihren Anlagestellen (26, 28) gegen die Anlagemittel (42) des Schneidklingenhalters bewirken.

8. Mikrokeratom nach einem der vorhergehenden Ansprüche,
**dadurch gekennzeichnet, dass** die Anlagemittel (42) des Schneidklingenhalters (10) eine bei Betrachtung in einem Schnitt quer zur Unearerstreckung der Anlagemittel bogenartig, insbesondere kreisbogenartig, gekrümmte Kontur besitzen.

## Claims

1. A microkeratom, in particular for refractive eye surgery, comprising a cutting blade holder (10) and a cutting blade unit (14) which can be inserted into an adaptor (34) of the cutting blade unit (14) so as to be laterally movable, with the cutting blade unit comprising a cutting blade (12) with a cutting edge (20) formed on a first blade margin, with the cutting blade holder comprising linear contact means (42) against which the cutting blade unit bears so that it is laterally movable in the properly inserted condition in the adaptor via a second blade margin (24) of the cutting blade opposite the first blade margin,
**characterised in that** the second blade margin of the cutting blade forms at least two spaced contact locations (26, 28) for the contact of the cutting blade with the contact means of the cutting blade holder and, between each pair of neighbouring contact locations, is recessed (at 30) towards the first blade margin with respect to an imaginary straight line which connects the respective contact locations.

2. The microkeratom according to Claim 1,
**characterised in that** at least one contact location (26, 28) of the cutting blade (12) is formed by a rounded portion of the second blade margin (24).

3. The microkeratom according to Claim 1 or 2,
**characterised in that** at least one contact location (26b, 28b) of the cutting blade (12b) is formed by a pointed portion of the second blade margin (24b).

4. The microkeratom according to one of the previous claims,
**characterised in that** at least one contact location (26a, 28a) of the cutting blade (12a) is formed by a straight portion of the second blade margin (24b).

5. The microkeratom according to one of the previous claims,
**characterised in that** at least two contact locations (26c, 50c) have different geometries.

6. The microkeratom according to one of the previous claims,
**characterised in that** the second blade margin (24c) of the cutting blade (12c) forms at least three contact locations (26c, 28c, 50c).

7. The microkeratom according to one of the previous claims,
**characterised in that** with the cutting blade unit (14) properly inserted in the cutting blade holder (10), resilient bias means (44) are effective between said cutting blade unit (14) and the cutting blade holder, which cause the cutting blade to be urged with its contact locations (26, 28) against the contact means (42) of the cutting blade holder.

8. The microkeratom according to one of the previous claims,
**characterised in that** the contact means (42) of the cutting blade holder (10), when viewed in a section transversely to the linear extension of the contact means, have an arc-shaped, in particular, circular arc-shaped curved contour.

## Revendications

1. Microkératome, en particulier pour la chirurgie oculaire réfractive, comprenant un porte-lame de coupe (10) et une unité de lame de coupe (14) pouvant être placée de manière latéralement mobile dans un logement (34) du porte-lame de coupe, l'unité de lame de coupe présentant une lame de coupe (12) avec une arête de coupe (20) réalisée sur premier un bord de lame et le porte-lame de coupe présentant des moyens d'appui linéaires (42) contre lesquels l'unité de lame de coupe, lorsqu'elle est correctement placée dans le logement, s'appuie de manière latéralement mobile par l'intermédiaire d'un deuxième bord de lame (24) opposé au premier bord de la lame de coupe,
**caractérisé en ce que** le deuxième bord de la lame de coupe forme au moins deux zones d'appui (26, 28) disposées à distance l'une de l'autre pour l'appui de la lame de coupe contre les moyens d'appui du porte-lame de coupe et est, entre chaque paire de zones d'appui voisines l'une de l'autre, en retrait (à 30) par rapport à une droite imaginaire reliant lesdites zones d'appui et en direction du premier bord de lame.

2. Microkératome selon la revendication 1,
**caractérisé en ce qu'**au moins une zone d'appui (26, 28) de la lame de coupe (12) est formée par un segment arrondi du deuxième bord de lame (24).

3. Microkératome selon la revendication 1 ou 2,
**caractérisé en ce qu'**au moins une zone d'appui (26b, 28b) de la lame de coupe (12b) est formée par un segment du deuxième bord de lame (24b) se terminant en pointe.

4. Microkératome selon l'une des revendications précédentes,
**caractérisé en ce qu'**au moins une zone d'appui (26a, 28a) de la lame de coupe (12a) est formée par un segment du deuxième bord de lame (24a) s'étendant en ligne droite.

5. Microkératome selon l'une des revendications précédentes,
**caractérisé en ce qu'**au moins deux zones d'appui (26c, 50c) possèdent une géométrie différente.

6. Microkératome selon l'une des revendications précédentes,
**caractérisé en ce que** le deuxième bord de lame (24c) de la lame de coupe (12c) forme au moins trois zones d'appui (26c, 28c, 50c).

7. Microkératome selon l'une des revendications précédentes,
**caractérisé en ce que**, lorsque l'unité de lame de coupe (14) est correctement placée dans le porte-lame de coupe (10), des moyens de précontrainte à élasticité de ressort (44) agissent entre ladite unité de lame de coupe et le porte-lame de coupe, lesquels moyens ont pour effet, aux zones d'appui (26, 28), de presser la lame de coupe contre les moyens d'appui (42) du porte-lame de coupe.

8. Microkératome selon l'une des revendications précédentes,
**caractérisé en ce que** les moyens d'appui (42) du porte-lame de coupe (10) possèdent un contour courbé en arc, en particulier en arc de cercle, vu en coupe transversale par rapport à l'étendue linéaire des moyens d'appui.
